# FASCICULE DE BREVET EUROPEEN

(11) **EP 4 555 067 B1**
(45) Date de publication et mention de la délivrance du brevet: **02.09.2026**
(21) Numéro de dépôt: 23748563.6
(22) Date de dépôt: 10.07.2023
(51) Int. Cl.: C12M 3/00, C12M 1/00, C12M 1/32, C12M 1/12

(54) **INSTALLATION DE CULTURE DE TISSU CELLULAIRE**
ANLAGE ZUR ZELLKULTUR VON ZELLENGEWEBE
FACILITY FOR CELLULAR TISSUE CULTURE

(30) Priorité: 13.07.2022 FR 2207248
(43) Date de publication de la demande: 21.05.2025
(73) Titulaire: Adhara, 75116 Paris (FR)
(72) Inventeur: VALOIS, Pascal, 21000 Dijon (FR); GIRARD, Mathilde, 21300 Chenove (FR); LIRSAC, Pierre-Noël, 67100 Strasbourg (FR); GARO, Ronan, 21000 Dijon (FR); ALLIOUCHE, Mérouane, 68200 Mulhouse (FR)
(74) Mandataire: Plasseraud IP
(86) Numéro de dépôt international: PCT/FR2023/051066
(87) Numéro de publication internationale: WO 2024/013449

(56) Documents cités:
- EP-A1- 3 782 729
- WO-A1-2014/120772
- WO-A1-2017/066821
- US-A1- 2010 304 472
- US-B2- 9 163 208

## Description

### Domaine technique

La présente divulgation relève des systèmes de culture de tissu cellulaire.

### Technique antérieure

Le processus de fabrication de tissu cellulaire, comme de la peau artificielle, implique l'utilisation d'un environnement clos et d'une pluralité de milieux de culture apportés à la culture de manière séquentielle. Le processus peut prendre du temps et impliquer beaucoup de manipulations du système clos par un opérateur.

Typiquement, des cellules sont mises dans une boîte de culture. Cette boîte de culture constitue un environnement stérile dans lequel les différentes couches de la peau vont être crées au fil d'apports de milieux de culture. À chaque fois qu'un milieu doit être fourni à la culture, l'opérateur débouchonne/dévisse/ouvre la boîte et introduit le milieu de culture. Puis il rebouchonne/revisse/referme la boîte, la repose et laisse le tout reposer. Il reprend la boîte, débouchonne, introduit, rebouchonne ainsi pour chaque milieu de culture.

D'une part les multiples opérations d'ouverture et de fermeture de la boîte peuvent amener à des contaminations du milieu de culture. D'autre part, cela requiert un large temps d'opérateur ce qui ne permet pas une multiplication à grande échelle de la culture de tissu cellulaire. WO2014/120772A1 fait partie de l'art antérieur et divulgue une installation de culture de tissu cellulaire (100) ayant un système de distribution (202, 204) comprenant un support (202, 204) adapté à recevoir au moins deux boîtes (206) de culture de tissu cellulaire et un premier réseau fluidique de distribution (414(1)-414(6)) connecté au support (18).

### Résumé

Il est proposé une installation de culture de tissu cellulaire qui comprend un système de distribution comprenant : un support adapté à recevoir au moins deux boîtes de culture de tissu cellulaire, un premier réseau fluidique de distribution connecté au support, le premier réseau fluidique de distribution étant adapté à amener un premier fluide de type biomatériau vers chacune des boîtes de culture de tissu cellulaire, le premier réseau fluidique de distribution ayant un premier port aseptique d'entrée sur le support, le premier port aseptique d'entrée étant un unique port d'entrée pour le premier fluide, un deuxième réseau fluidique de distribution connecté au support, le deuxième réseau fluidique de distribution étant adapté à amener un deuxième fluide vers chacune des boîtes de culture de tissu cellulaire, le deuxième réseau fluidique de distribution ayant un deuxième port aseptique d'entrée sur le support différent du premier port aseptique d'entrée, le deuxième port aseptique d'entrée étant un unique port d'entrée pour le deuxième fluide, un troisième réseau fluidique de distribution connecté au support, le troisième réseau fluidique de distribution étant adapté à vidanger un troisième fluide de chacune des boîtes de culture de tissu cellulaire, le troisième réseau fluidique de distribution ayant un unique port aseptique de sortie sur le support, et un système d'alimentation connecté au système de distribution, le système d'alimentation comprenant : un premier réseau fluidique d'alimentation connecté aseptiquement au premier réseau fluidique de distribution via le premier port aseptique d'entrée, le premier réseau fluidique d'alimentation inclut une connexion aseptique adaptée à connecter avec une première poche lors de leur mélange dans le mélangeur statique polymérisent pour former le premier fluide, le premier réseau fluidique d'alimentation incluant une pompe adaptée à amener le premier fluide vers le premier réseau fluidique de distribution, un deuxième réseau fluidique d'alimentation connecté aseptiquement au deuxième réseau fluidique de distribution via le deuxième port aseptique d'entrée, le deuxième réseau fluidique optionnel d'alimentation inclut une connexion aseptique adaptée à connecter avec une deuxième poche contenant le deuxième fluide, un deuxième réseau fluidique d'alimentation incluant une pompe adaptée à amener le deuxième fluide vers le deuxième réseau fluidique de distribution, et un troisième réseau fluidique d'alimentation connecté aseptiquement au troisième réseau fluidique de distribution via le port aseptique de sortie, le troisième réseau fluidique d'alimentation inclut une connexion aseptique adaptée à connecter avec une poche de vidange, le troisième réseau fluidique d'alimentation incluant une pompe adaptée à vidanger le troisième fluide du troisième réseau fluidique de distribution.

Les caractéristiques exposées dans les paragraphes suivants peuvent, optionnellement, être mises en oeuvre, indépendamment les unes des autres ou en combinaison les unes avec les autres :
- le support inclut de plus un système de vidange connecté à au moins un des du premier et optionnellement deuxième réseaux fluidiques de distribution.
- chacun des premier, optionnellement deuxième et troisième réseaux fluidiques de distribution comprend une connexion aseptique adaptée à connecter chacun des premier, optionnellement deuxième et troisième réseaux fluidiques de distribution aux boîtes de culture de tissu cellulaire.
- chacun des premier, optionnellement deuxième et troisième réseaux fluidiques de distribution inclut un tube principal et deux tubes secondaires, le tube principal étant d'une part connecté au port aseptique et d'autre part connecté aux tubes secondaires, chacun des tubes secondaires étant connecté à une boîte de culture de tissu cellulaire associée.
- les pompes de chacun des premier, deuxième et troisième réseau fluidique d'alimentation est une pompe péristaltique.
- l'installation comprend de plus un système de rinçage connecté au premier ou deuxième réseau fluidique d'alimentation.
- le premier réseau fluidique d'alimentation comprend un mélangeur statique adapté à produire le premier fluide après passage dans le mélangeur statique.
- la connexion aseptique connecte avec deux poches contenant chacune un composant fluide qui se polymérisent lors du mélange par le mélangeur statique pour former le premier fluide.
- le système de distribution est un premier système de distribution, et comprenant de plus un deuxième système de distribution, le deuxième système de distribution ayant deux ports d'entrée aseptique et un port de sortie aseptique sur le support, chacun des premier, deuxième et troisième réseaux fluidiques d'alimentation ayant une première connexion aseptique au premier système de distribution et une deuxième connexion aseptique au deuxième système de distribution.
- l'installation comprend de plus un support individuel mécanisé pour chaque boîte, le support individuel mécanisé permettant la mise en mouvement des boîtes afin d'effectuer une agitation transversale mais également une inclinaison.
- le support inclut des moyens de connexion amovible desdites au moins deux boîtes de culture de tissu cellulaire sur le support.
- le système de vidange comprend une poche de vidange connectée à chacun des du premier réseau fluidique et du deuxième réseau fluidique de distribution.
- chacun des premier, optionnellement deuxième et troisième réseaux fluidiques de distribution comprend une connexion aseptique adaptée à connecter chacun des premier, deuxième et troisième réseau fluidique de distribution aux boîtes de culture de tissu cellulaire.
- le premier réseau fluidique de distribution inclut un tube principal et deux tubes secondaires, le tube principal étant d'une part connecté au port d'entrée et d'autre part connecté aux tubes secondaires, chacun des tubes secondaires ayant une extrémité connectée à une boîte de culture de tissu cellulaire associée de sorte à amener le premier fluide vers chacune des boîtes de culture de tissu cellulaire.
- le deuxième réseau fluidique optionnel de distribution inclut un tube principal et deux tubes secondaires, le tube principal étant d'une part connecté au port d'entrée et d'autre part connecté aux tubes secondaires, chacun des tubes secondaires étant connecté à une boîte de culture de tissu cellulaire associée de sorte à amener le deuxième fluide vers chacune des boîtes de culture de tissu cellulaire.
- le troisième réseau fluidique de distribution inclut un tube principal et deux tubes secondaires, le tube principal étant d'une part connecté au port d'entrée et d'autre part connecté aux tubes secondaires, chacun des tubes secondaires ayant une extrémité connectée à une boîte de culture de tissu cellulaire associée de sorte à vider le troisième fluide des boîtes de culture de tissu cellulaire.
- dans lequel l'installation comprend de plus un système d'alimentation connecté au système de distribution, le système d'alimentation comprenant :un premier réseau fluidique d'alimentation connecté aseptiquement au premier réseau fluidique de distribution, le premier réseau fluidique d'alimentation incluant une pompe adaptée à amener le premier fluide vers le premier réseau fluidique de distribution, un deuxième réseau fluidique optionnel d'alimentation connecté aseptiquement au deuxième réseau fluidique de distribution, le deuxième réseau fluidique d'alimentation incluant une pompe adaptée à amener le deuxième fluide vers le deuxième réseau fluidique de distribution, et un troisième réseau fluidique d'alimentation connecté aseptiquement au troisième réseau fluidique de distribution, le troisième réseau fluidique d'alimentation incluant une pompe adaptée à vidanger le troisième fluide du troisième réseau fluidique de distribution.
- le premier réseau fluidique d'alimentation inclut une connexion aseptique adaptée à connecter avec une première poche contenant le premier fluide, le deuxième réseau fluidique d'alimentation inclut une connexion aseptique adaptée à connecter avec une deuxième poche contenant le deuxième fluide, et le troisième réseau fluidique d'alimentation inclut une connexion aseptique adaptée à connecter avec une poche de vidange.
- chacun des premier, optionnellement deuxième et troisième réseau fluidique d'alimentation comprend au moins une pompe, préférentiellement une pompe péristaltique ou tout autre système permettant de mettre en mouvement le fluide, tel que, par exemple, un pousse-seringue.
- le système d'alimentation inclut au moins un filtre.
- ledit au moins un filtre est fluidiquement connecté à au moins un des premier et deuxième réseau fluidique d'alimentation par un canal d'air.
- une connexion entre le canal d'air et le deuxième réseau fluidique inclut un système de fermeture pouvant être, par exemple, une vanne, une électrovanne ou un système de pincement de tube.
- le système d'alimentation comprend de plus un système de rinçage connecté au premier ou deuxième réseau fluidique d'alimentation.
- le premier réseau fluidique d'alimentation inclut un tube principal et deux tubes secondaires, le tube principal étant d'une part connecté au système de distribution et d'autre part connecté aux tubes secondaires, chacun des tubes secondaires ayant une extrémité adaptée à être connectée à une poche contenant un fluide.
- le système d'alimentation comprend de plus chacune des poches connectées aux tubes secondaires, une des poches contenant chacune du plasma et l'autre une solution saline, ou bien une des poches contenant chacune du collagène et l'autre du DMEM (medium modifiable de Dulbecco).
- le tube principal est connecté aux deux tubes secondaires par un mélangeur statique, par exemple un connecteur Y, adapté à produire le premier fluide après passage dans le mélangeur statique.
- chacun des tubes secondaires comprend une pompe, de préférence une pompe péristaltique.
- le premier réseau fluidique d'alimentation comprend un mélangeur statique adapté à produire le premier fluide après passage dans le mélangeur statique.
- le système d'alimentation comprend de plus au moins un système de fermeture tel qu'un système à pincement de tubes.
- ledit au moins système de fermeture inclut un premier système de pincement de tube sur le premier réseau fluidique et/ou un deuxième système de pincement de tube sur un réseau fluidique connectant un système de rinçage au deuxième réseau fluidique d'alimentation.
- le système de distribution est un premier système de distribution, et comprenant de plus un deuxième système de distribution, le deuxième système de distribution ayant deux ports d'entrée aseptique et un port de sortie aseptique sur le support,
   chacun des premier, deuxième et troisième réseaux fluidiques d'alimentation ayant une première connexion aseptique au premier système de distribution et une deuxième connexion aseptique au deuxième système de distribution.
- le système d'alimentation comprend de plus une deuxième poche contenant le deuxième fluide connectée au deuxième réseau fluidique d'alimentation, et une troisième poche vide connectée au troisième réseau fluidique d'alimentation.
- la deuxième poche comprend un agent de conservation qui peut être un cryopréservant, par exemple du DMSO (sulfoxide de diméthyl).

### Brève description des dessins

D'autres caractéristiques, détails et avantages apparaîtront à la lecture de la description détaillée ci-après, et à l'analyse des dessins annexés, sur lesquels :
Fig. 1

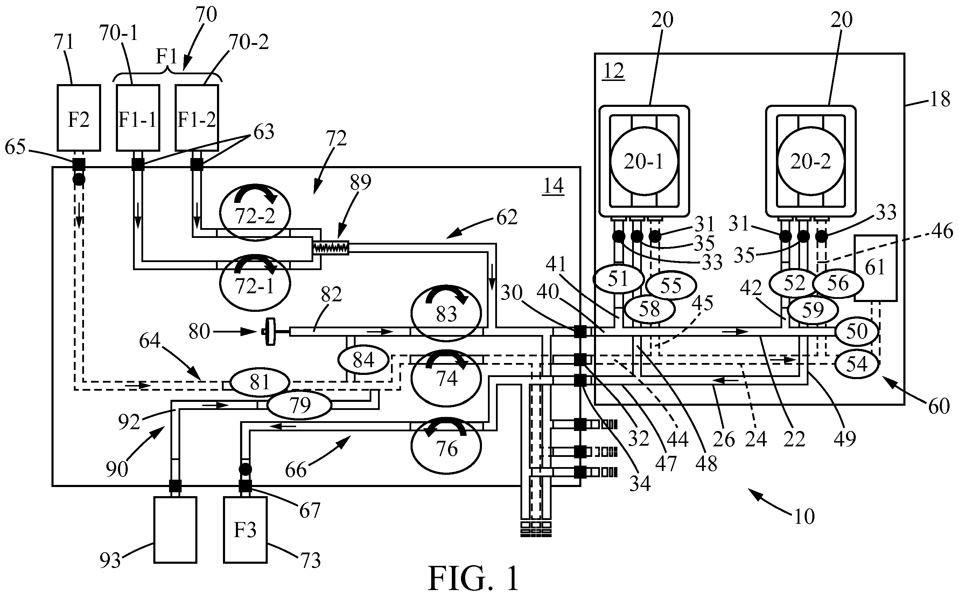

   La [Fig. 1] est une vue schématique d'une installation de culture de tissu cellulaire ;
Fig. 2

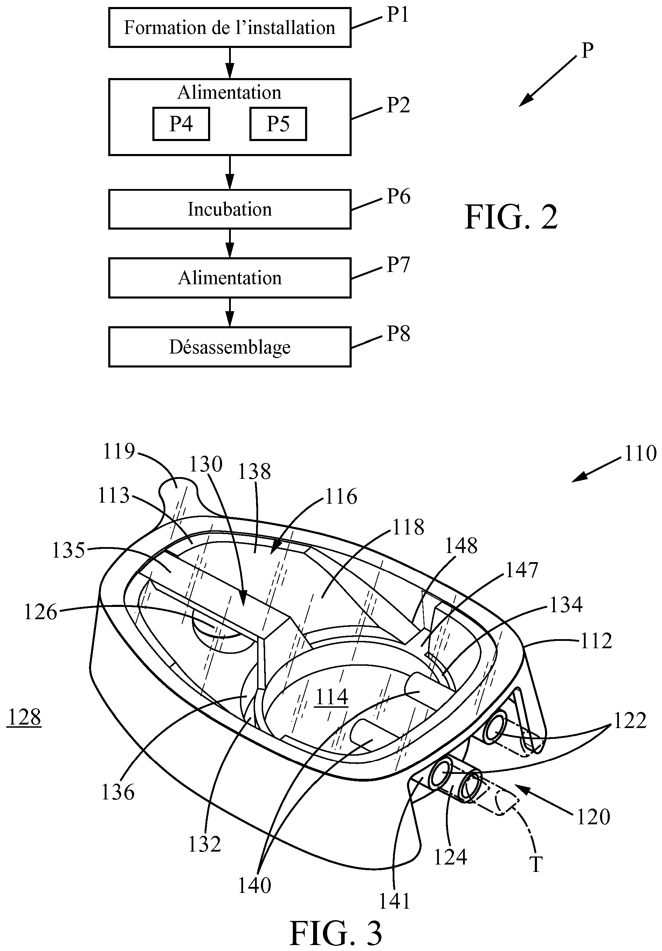

   La [Fig. 2] est un ordinogramme illustrant le procédé d'opération de l'installation de culture de tissu cellulaire de la figure 1 ;
Fig.3
   La [Fig. 3] est une vue schématique en perspective du dessus d'une boîte de culture et transport de tissu cellulaire selon un mode de réalisation, utilisable avec l'installation de culture de tissu cellulaire de la figure 1 ;
Fig. 4

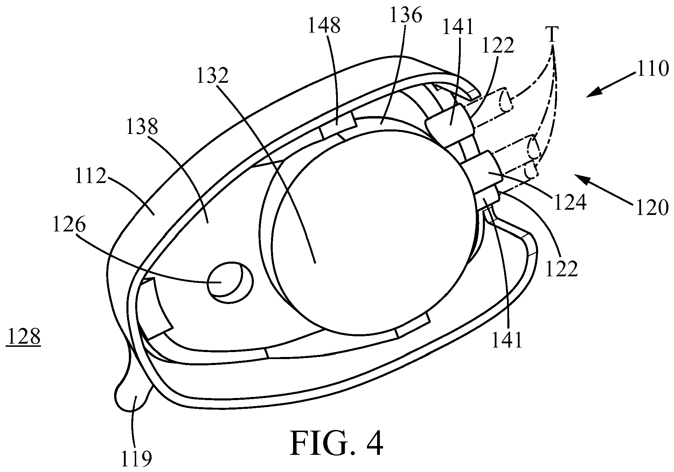

   La [Fig. 4] est une vue schématique en perspective du dessous de la boîte de la figure 3;
Fig. 5

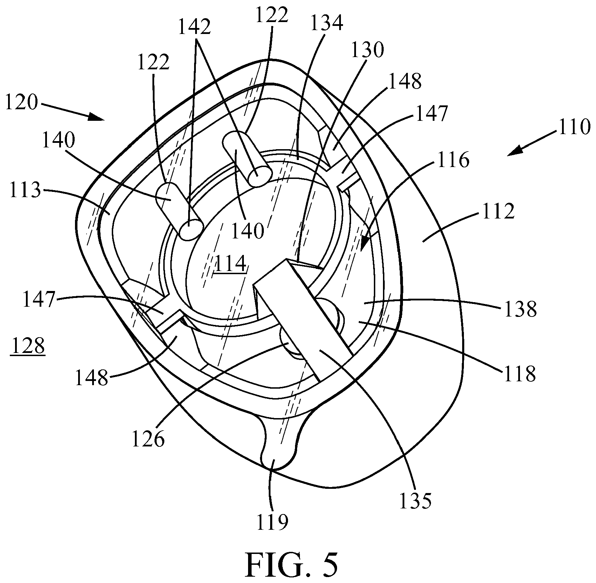

   La [Fig. 5] est une autre vue schématique en perspective du dessus de la boîte de la figure 3;
Fig. 6

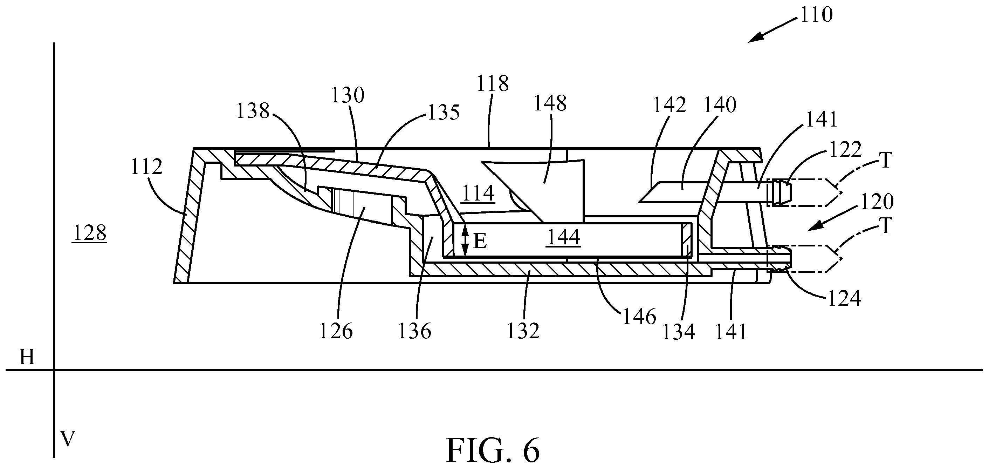

   La [Fig. 6] est une vue schématique en coupe de la boîte de la figure 3;
Fig. 7

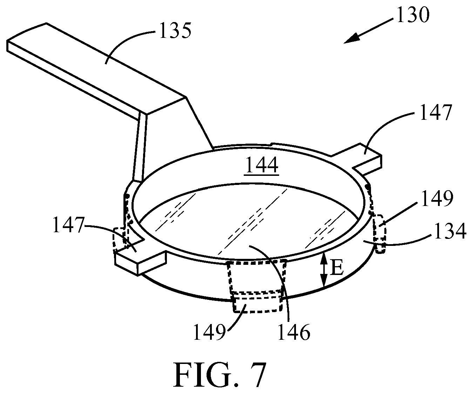

   La [Fig. 7] est une vue schématique en perspective d'un insert pour la boîte de la figure 3 ; et
Fig. 8

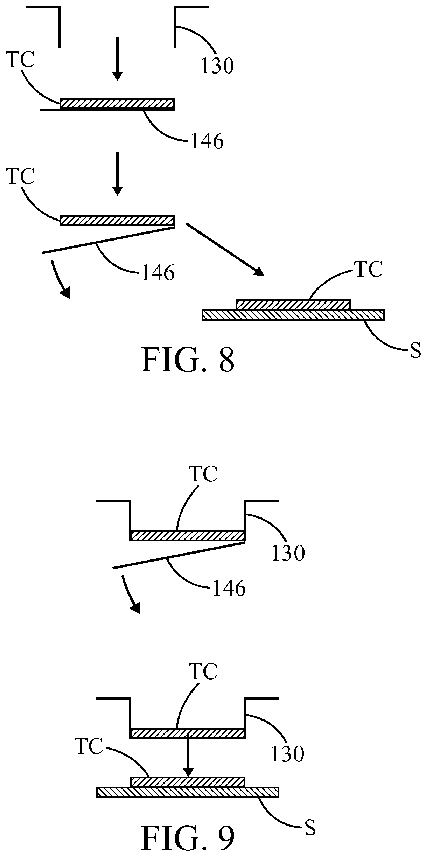

   La [Fig. 8] est une vue schématique d'un mode de réalisation de l'insert où un fond poreux de l'insert est détachable ;
Fig. 9
   La [Fig. 9] est une vue schématique d'un autre mode de réalisation de l'insert où un fond poreux de l'insert est détachable ;
Fig. 10

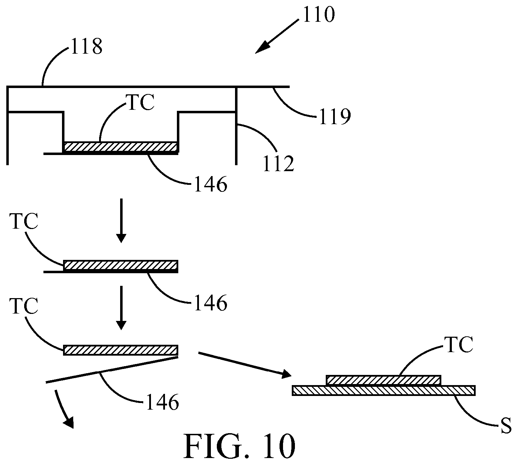

   La [Fig. 10] est une vue schématique d'un mode de réalisation de la boîte où la boîte a un fond amovible ; et
Fig. 11

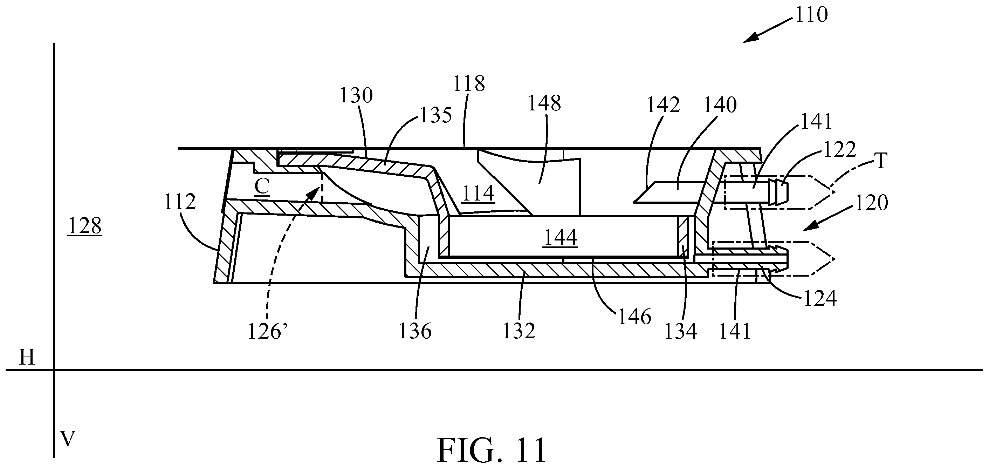

   La [Fig. 11] est une vue schématique d'un autre mode de réalisation de la boîte où un filtre est disposé verticalement.
**Fig. 12**

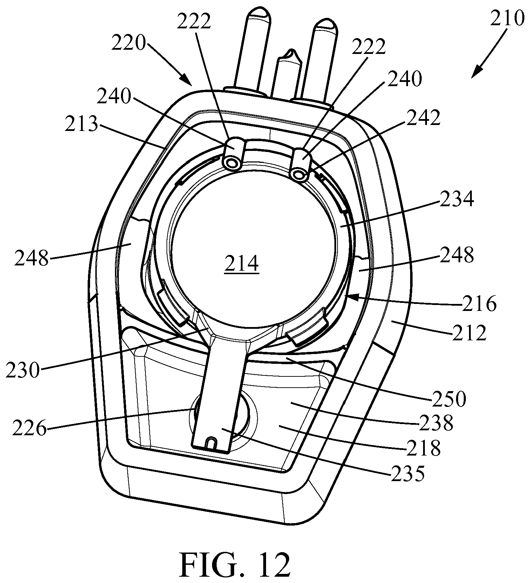

   La [Fig. 12] est une vue schématique d'un autre mode de réalisation de la boîte qui inclut une cloison entre le filtre et la zone de travail.

### Description des modes de réalisation

Il est maintenant fait référence à la figure 1, une installation de culture de tissu cellulaire 10 va être décrite. L'installation 10 a pour but de cultiver plusieurs tissus cellulaires en même temps afin d'augmenter la production de tels tissus. De plus, la culture se fait en milieu clos, fermé, stérile ce qui permet de réduire les risques de contamination. Grâce à son système clos, l'installation 10 a pour but d'être utilisée dans un environnement qui ne nécessiterait pas de poste de sécurité microbiologique. La culture peut se faire enfin avec un apport minimal de l'opérateur. Pour ce faire, l'opération de l'installation peut être automatisée. L'installation est à usage unique. Ainsi, une fois la culture cellulaire achevée, tous les tuyaux et connecteurs et autres éléments de l'installation peuvent être dissociés et mis de cotés (possiblement jetés), et les boîtes peuvent être transportées jusqu'à leur destination d'utilisation. Elles peuvent être cryogénées pour préverser le tissu cultivé se trouvant dans chaque boite pendant le transport.

L'installation 10 comprend un système de distribution 12 où une pluralité de boîtes 20 de culture de tissu cellulaire peuvent être disposées et un système d'alimentation 14 connectable au système de distribution 12 et capable d'alimenter les boîtes 20 afin de réaliser une culture de tissu cellulaire dans chacune des boîtes 20. Les systèmes de distribution 12 et alimentation 14 comprennent plusieurs réseaux fluidiques de sorte à relier des poches de milieux de culture aux boîtes 20 et permettre leur alimentation/drainage, le tout en restant en système clos pour prévenir toute contamination. L'installation 10 permet ainsi de réaliser des tissus cellulaires dans plusieurs groupes de boîtes en même temps en utilisant une même alimentation en milieux de culture. Les réseaux fluidiques connectés en permanence aux boîtes 20 et aux milieux de culture permettent de diminuer des risques de contamination et diminuent le temps de production, puisque les manipulations des boîtes sont diminuées. Le système d'alimentation 14 permet d'alimenter un ou plusieurs systèmes de distribution 12, chacun desquels comporte plusieurs boîtes 20 de culture. Une production en lots de tissus cellulaires peut ainsi être accomplie.

Le système de distribution 12 comprend un (ou plusieurs) support 18 adapté à recevoir au moins deux boîtes 20 de culture de tissu cellulaire. Dans l'exemple de la figure 1, deux boîtes 20-1 et 20-2 sont illustrées. Il pourrait y avoir plus que deux boîtes sur le support 18, par exemple un support 18 pourrait contenir 10 boîtes 20. Un exemple de boîte 20 sera donné plus bas. Les boîtes de culture cellulaire 20 peuvent être amovibles du support 18. Le support 18 selon un mode de réalisation peut être réutilisable avec plusieurs boîtes 20. Le support 18 peut recevoir par exemple jusqu'à dix boîtes 20. Le nombre de boîtes 20 et le nombre de supports 18 utilisés dans le système de distribution 12 peut dépendre des volumes de milieux de culture disponible pour la culture, de la taille de l'installation finale et du temps d'acheminement des fluides si celui-ci est trop long pour un nombre trop important de supports 18 alimentés en parallèles. Le support 18 peut être adapté à être mis dans un incubateur. En ce sens, la taille du support 18 peut être ajustée pour permettre sa mise sous incubation.

Chaque boîte 20 est connectable à un premier réseau fluidique de distribution 22 adapté à lui amener un premier fluide F1, par exemple un biomatériau, à un deuxième réseau fluidique de distribution 24 adapté à lui amener un ou plusieurs deuxième fluide F2, par exemple un ou plusieurs milieux de culture acheminés de façon séquentielle, et à un troisième réseau fluidique de distribution 26, adapté à vidanger d'elle un troisième fluide F3. Chacun des premier 22, deuxième 24 et troisième 26 réseaux fluidiques de distribution sont connectés au support 18, de façon amovible ou pas. Les réseaux fluidiques 22, 24, 26 sont typiquement des tubes plastiques connectés entre eux de façon à créer des branchements.

Le premier réseau fluidique de distribution 22 a un unique port de connexion aseptique 30 au système d'alimentation 14 pour permettre l'acheminement du premier fluide F1, en l'occurrence un port d'entrée aseptique, et autant de ports de connexion aseptiques 31 en sortie que de boîtes 20 sur le support 18 (un port de sortie aseptique 31 par boîte 20).

Le deuxième réseau fluidique de distribution 24 a un unique port de connexion aseptique 32 au système d'alimentation 14 pour permettre l'acheminement du ou des deuxième F2, en l'occurrence un port d'entrée aseptique, et autant de ports de connexions aseptiques 33 en sortie que de boîtes 20 sur le support 18 (un port de sortie aseptique 33 par boîte 20).

Le troisième réseau fluidique de distribution 26 a un unique port de connexion aseptique 34 au système d'alimentation 14, en l'occurrence un port de sortie aseptique pour évacuer les fluides des boîtes 20, et autant de ports de connexion aseptiques 35 en entrée que de boîtes 20 sur le support 18 (un port d'entrée aseptique 35 par boîte 20).

Chaque boîte 20 est connectée aux réseaux fluidiques 22, 24, 26 par connexion aseptique (le tube peut être emmanché, collé ou soudé). Les boîtes 20 seront alors déconnectées des réseaux fluidiques 22, 24, 26 par soudure puis coupure des tubes. Dans ce cas, une petite partie des tubes peut rester sur chaque boîte 20 pour maintenir l'étanchéité dans la boîte 20 lorsque l'utilisation de l'installation 10 est terminée.

Du fait que chacun des premier, deuxième et troisième réseau fluidique 22, 24, 26 a un unique port de connexion au système d'alimentation 14 et de multiples ports de connexion aux boîtes, les réseaux fluidiques 22, 24, 26 sont en arborescence avec un tube principal s'étendant à partir de chacun des ports de connexion 30, 32, 34 et des tubes secondaires émanant de chacun des tubes principaux. Les tubes des réseaux fluidiques sont par exemple des tuyaux de PVC, EVA (éthylène-acétate de vinyle) ou silicone compatibles avec des pompes, électrovannes, et la cryopréservation. Les réseaux fluidiques 22, 24, 26 peuvent être constitués de différents types et/ou tailles de tubes.

Ainsi, le premier réseau fluidique 22 de distribution inclut un tube principal 40 et deux tubes secondaires 41, 42. Le tube principal 40 d'une part connecté au port de connexion aseptique 30, et d'autre part connecté aux tubes secondaires 41, 42. Chacun des tubes secondaires 41, 42 a une extrémité connectée à une boîte 20 de culture de tissu cellulaire associée (i.e. aux ports de connexion aseptiques 31) de sorte à amener le premier fluide F1 vers chacune des boîtes 20 de culture de tissu cellulaire.

Le deuxième réseau fluidique 24 de distribution inclut un tube principal 44 et deux tubes secondaires 45, 46. Le tube principal 44 est d'une part connecté au port de connexion aseptique 32, et d'autre part connecté aux tubes secondaires 45, 46. Chacun des tubes secondaires 45, 46 ayant une extrémité connectée à une boîte 20 de culture de tissu cellulaire associée (i.e. aux ports de connexion aseptiques 33) de sorte à amener le ou les deuxième fluide F2 vers chacune des boîtes 20 de culture de tissu cellulaire.

Le troisième réseau fluidique 26 de distribution inclut un tube principal 47 et deux tubes secondaires 48, 49. Le tube principal 47 est d'une part connecté au port de connexion aseptique 34, et d'autre part connecté aux tubes secondaires 48, 49. Chacun des tubes secondaires 48, 49 a une extrémité connectée à une boîte 20 de culture de tissu cellulaire associée de sorte à vider le troisième fluide F3 des boîtes 20 de culture de tissu cellulaire.

Chacun des tubes secondaires 41, 42, 45, 46, 48, 49 peut respectivement inclure un système de fermeture 51, 52, 55, 56, 58, 59. Les systèmes de fermeture 51, 52, 55, 56, 58, 59 peuvent être par exemple des systèmes à pincement de tubes permettent de contrôler le débit de fluide allant dans chacune des boîtes 20. Ainsi, il est possible d'alimenter séquentiellement les boîtes 20 en fluides, en ouvrant ou fermant sélectivement les systèmes de fermeture ou vannes 51, 52, 55, 56, 58, 59. Les vannes 51, 52, 55, 56, 58, 59 permettent que chaque boîte 20 reçoive la même quantité de liquide. En effet, comme les boîtes 20 sont alimentées en série dans le système d'alimentation 12, il se pourrait qu'une boîte 20 en amont de l'écoulement reçoive plus de fluide qu'une boîte se trouvant en aval. Les vannes 51, 52, 55, 56, 58, 59 sont un exemple de système de régulation du débit. Les vannes 51, 52, 55, 56, 58, 59 peuvent être n'importe quel système permettant de réguler le débit, comme un pincement mécanique, ou une valve classique. L'avantage des systèmes de pincement est que le pincement est extérieur au tube, et donc sans contact avec le milieu de culture, ce qui diminue les risques de contamination. Les systèmes de fermeture 51, 52, 55, 56, 58, 59 pourraient être des systèmes de pincement de type vanne, comme des électrovannes ou des systèmes avec une came et un moteur ou pneumatique. Les systèmes de fermeture 51, 52, 55, 56, 58, 59 peuvent être actionnés de façon manuelle, ou bien opéré par une unité de contrôle, de sorte que le système soit partiellement ou entièrement automatique.

Le support 18 peut inclure de plus un système de vidange 60 connecté à au moins un des premier 22 et deuxième 24 réseaux fluidiques de distribution. Dans le mode de réalisation illustrée en figure 1, le système de vidange 60 est connecté à chacun des premier 22 et deuxième 24 réseaux fluidiques de distribution. Selon un mode de réalisation, le système de vidange 60 comprend une poche de vidange 61 connectée à chacun des tubes principaux 40 et 44. La poche de vidange 61 sert à récupérer une partie du premier fluide F1 et du deuxième fluide F2 (en l'occurrence l'amorçage du mélange du biomatériau), ainsi qu'à la récupération d'un liquide de rinçage provenant du deuxième réseau fluidique de distribution 24 lorsque le deuxième réseau fluidique de distribution 24 est nettoyé entre différents fluides F2. Les différents deuxièmes fluides F2 qui représentent des milieux de culture peuvent par exemple être du plasma et de la solution saline, ou bien du collagène et du DMEM (medium modifiable de Dulbecco). Un des deuxièmes fluide F2 peut aussi être un cryopréservant, par exemple du DMSO (sulfoxide de diméthyl). Les tubes principaux 40 et 44 peuvent chacun inclure une vanne 50, 54, par exemple une électrovanne, pour contrôler l'accès à la poche de vidange 61. Selon un mode de réalisation, la poche de vidange 61 n'est pas commune aux premier 22 et deuxième 24 réseaux fluidiques de distribution, et chacun des premier 22 et deuxième 24 réseaux fluidiques de distribution inclut sa poche de vidange connectée respectivement aux vannes 50, 54 des tubes principaux 40 et 44.

En se focalisant maintenant sur le système d'alimentation 14, celui-ci comprend un premier réseau fluidique d'alimentation 62 connecté aseptiquement au premier réseau fluidique de distribution 22 au port de connexion aseptique 30, un deuxième réseau fluidique d'alimentation 64 connecté aseptiquement au deuxième réseau fluidique de distribution 24 au port de connexion aseptique 32, et un troisième réseau fluidique d'alimentation 66 connecté aseptiquement au troisième réseau fluidique de distribution 66 au port de connexion aseptique 34. Le premier réseau fluidique d'alimentation 62 inclut un port de connexion aseptique 63 adaptée à se connecter avec une première poche 70 contenant le premier fluide F1. Le deuxième réseau fluidique d'alimentation 64 inclut un port de connexion aseptique 65 adaptée à se connecter avec une deuxième poche 71 contenant le deuxième fluide F2. Le troisième réseau fluidique d'alimentation 66 inclut un port de connexion aseptique 67 adaptée à se connecter avec une poche de vidange 73.

Le premier réseau fluidique d'alimentation 62 inclut une pompe 72 adaptée à amener le premier fluide F1 de la première poche 70 vers le premier réseau fluidique de distribution 22. Le deuxième réseau fluidique d'alimentation 64 inclut une pompe 74 adaptée à amener le deuxième fluide F2 de la deuxième poche 71 vers le deuxième réseau fluidique de distribution 24. Le troisième réseau fluidique d'alimentation 66 inclut une pompe 76 adaptée à vidanger le troisième fluide F3 du troisième réseau fluidique de distribution 26. Les pompes 72, 74, 76 sont préférentiellement des pompes péristaltiques. Les pompes 72, 74, 76 pourraient être tout système permettant de mettre en mouvement le fluide, tel que, par exemple, un pousse-seringue.

Dans l'exemple de la figure 1, le premier fluide F1 est un biomatériau sous forme de gel. Le biomatériau est obtenu par le mélange de deux composants fluides F1-1 et F1-2, qui polymériseront lors de leur mélange. Le premier réseau fluidique d'alimentation 62 est donc relié à deux poches 70-1 et 70-2 différentes qui ensemble forment la poche 70. Les fluides F1-1 et F1-2 se rejoignent à un mélangeur statique 89, par exemple un connecteur Y. Comme le premier fluide F1 est obtenu par le mélange des fluides F1-1 et F1-2, la pompe 72 est l'ensemble de deux pompes 72-1 et 72-2 chacune sur un tube reliant la poche correspondante 70-1 et 70-2 au mélangeur statique 89. Chacune des pompes 72-1 et 72-2 peut être une pompe péristaltique.

Le système d'alimentation 14 peut inclure de plus un (ou plusieurs) filtre 80. Le filtre 80 permet de purifier l'air apporté dans le système d'alimentation 14. Le (ou les) filtre 80 est fluidiquement connecté à au moins un des premier 62 et deuxième 64 réseau fluidique d'alimentation par un canal d'air 82. Le filtre 80 est fluidiquement connecté au premier 62 et/ou au deuxième 64 réseau fluidique d'alimentation. Dans le mode de réalisation des figures, le filtre 80 est fluidiquement connecté au premier 62 réseau fluidique d'alimentation en aval d'une pompe 83, par exemple une pompe péristaltique, et au deuxième 64 réseau fluidique d'alimentation en amont de la pompe 83. Selon un autre mode de réalisation, le canal d'air 82 est seulement connecté au deuxième réseau fluidique d'alimentation 64 et l'installation 10 n'a pas la pompe 83. Une connexion entre le canal d'air 82 et le deuxième réseau fluidique d'alimentation 62 peut inclure une électrovanne 84. Selon un mode de réalisation, le deuxième réseau fluidique d'alimentation 62 inclut son propre canal d'air, de sorte que la connexion entre le canal d'air 82 et le deuxième réseau fluidique d'alimentation 62 n'a plus lieu d'être.

Le système d'alimentation 14 peut inclure de plus un système de rinçage 90 connecté au deuxième réseau fluidique d'alimentation 64. Le système de rinçage 90 permet de nettoyer le deuxième réseau fluidique d'alimentation 64 entre chaque deuxième fluide F2, si le deuxième réseau fluidique d'alimentation 64 est utilisé pour acheminer différents fluides F2 nécessaires pour la culture de tissu cellulaire. Le système de rinçage 90 inclut une poche d'alimentation du liquide de rinçage 93 et un réseau fluidique 92 connectant la poche d'alimentation du liquide de rinçage 93 au deuxième réseau fluidique d'alimentation 64. Le réseau fluidique 92 peut inclure une vanne 79, par exemple une électrovanne, pour sélectivement bloquer l'accès à la poche d'alimentation du liquide de rinçage 93. Une vanne 81 sur le deuxième réseau fluidique d'alimentation 64 en amont de la pompe 74 permet d'utiliser le système de rinçage 90 sans contamination de la poche 71, comme il sera expliqué plus bas.

Le système d'alimentation 14 peut inclure un ou plusieurs capteurs (non illustrés) qui permettraient de détecter les liquides et/ou les bulles d'air afin de détecter des défauts dans l'installation. Selon un exemple, un détecteur de bulles est positionné sur le premier réseau fluidique d'alimentation 62 en aval du mélangeur 89. Selon un autre exemple, un détecteur de fluide est positionné sur le deuxième réseau fluidique d'alimentation 64 en aval de la vanne 81.

Tel que montré à la figure 1 en pointillés, un même système d'alimentation 14 peut alimenter plusieurs systèmes de distribution 14 en même temps. Chaque système de distribution a ainsi deux ports d'entrée aseptique et un port de sortie aseptique sur le support de sorte à connecter au système d'alimentation 12.

La (et les) boîte(s) 20 peu(ven)t être n'importe quelle boîte de culture et transport de tissu cellulaire constituant un système fermé et ayant au moins deux connexions fluidiques d'entrée de fluides et une connexion fluidique de sortie de fluides, afin de se connecter au premier 22, deuxième 24 et troisième 26 réseaux fluidiques de distribution. De façon plus particulière, la boîte 20 comporte un corps de boîte ayant un intérieur accessible et une ouverture supérieure. Un filtre est disposé dans le corps de boîte et communiquant l'intérieur du corps de boîte avec un extérieur du corps de boîte. Le filtre est configuré pour permettre un échange gazeux entre l'intérieur et l'extérieur du corps de boîte tout en empêchant une contamination de la culture de tissu cellulaire. Un opercule ferme l'ouverture supérieure du corps de boîte. L'opercule est étanche aux fluides et aux gaz.

### Procédé de fonctionnement

Un procédé P de culture de tissu cellulaire va maintenant être décrit.

Dans une première étape P1 de formation de l'installation 10, les boîtes 20 de culture sont fixées à chaque support 18 si cela n'est pas déjà fait. La fixation comporte aussi la connexion des boîtes 20 aux réseaux fluidiques de distribution 22, 24 et 26. Les boîtes 20 sont vides. Selon un exemple, les boîtes 20 pourraient contenir un ou plusieurs composants nécessaires pour démarrer la culture de tissu cellulaire avant l'ajout des fluides de culture. Le système de distribution 14 est aussi connecté au système d'alimentation 12 en connectant les réseaux fluidiques de distribution 22, 24 et 26 aux réseaux fluidiques d'alimentation 62, 64, 66 via les connexions aseptiques 30, 32, 34. Le système de distribution 14 peut être placé dans un incubateur, ayant par exemple les conditions de 37°C, 5-6% CO2, 95% humidité tout en restant connecté au système d'alimentation 12.

Dans une deuxième étape P2 d'alimentation en fluides, une fois que toutes les connexions fluidiques sont effectuées, les fluides vont être acheminés séquentiellement aux boîtes.

Dans un premier temps, à l'étape P4 d'alimentation en premier fluide F1, les poches F1-1 et F1-2 sont connectées au premier réseau d'alimentation 62 à la connexion 63. Le premier fluide F1 va être acheminé dans chaque boîte 20 du support 18 de façon séquentielle afin d'optimiser le remplissage. Selon un mode de réalisation, une partie initiale du premier fluide F1 est d'abord purgé avant d'être acheminé aux boîtes 20. Ainsi, les vannes 51 et 52 sont fermées, et la vanne 50 ouverte. Les pompes 72-1 et 72-2 sont mises en route, ce qui permet au premier fluide F1 de passer des poches F1-1 et F1-2 dans le premier réseau d'alimentation 62. Sous l'action des pompes 72-1 et 72-2, les fluides F1-1 et F1-2 arrivent dans le mélangeur 89 puis dans le premier réseau de distribution 22, avant d'arriver dans la poche de vidange 61. Après un court instant, la vanne 50 est fermée puis la vanne 51 ouverte afin que le premier fluide F1 s'achemine maintenant dans la première boîte 20-1. Le flux de fluides F1-1 et F1-2 dans la première boîte 20-1 dure le temps nécessaire pour remplir une zone de culture cellulaire de la boîte 20-1. Une fois la première boîte 20-1 alimentée en premier fluide F1, la vanne 51 est fermée, puis la vanne 52 ouverte, afin que le premier fluide F1 s'achemine maintenant dans la deuxième boîte 20-2. Une fois toutes les boîtes 20 remplies, les pompes 72-1 et 72-2 sont arrêtées. La séquence pourrait être effectuée dans un autre sens. Par exemple en remplissant la boîte 20-2 avant la boîte 20-1. Selon un mode de réalisation, à l'issue de l'étape P4, le premier réseau d'alimentation 62 et le premier réseau fluidique 22 de distribution sont démantelés des boîtes et du support 18 (vu qu'ils ne serviront plus pour le reste du procédé).

Dans une étape P5 d'alimentation en deuxième fluide F2, un premier milieu de culture est acheminé aux boîtes 20. Ainsi, la poche 71 de deuxième fluide F2 est connectée au deuxième réseau d'alimentation 64 via la connexion aseptique 65. La vanne 81 est ouverte et la pompe 74 mise en route. La vanne 82 est fermée. Un premier jet de deuxième fluide F2 est purgé avant que les boîtes 20 reçoivent le deuxième fluide F2. De ce fait, la vanne 54 est ouverte et les vannes 55 et 56 sont fermées. Sous l'action de la pompe 74 le deuxième fluide F2 est acheminé de la poche 71 à la poche de vidange 61. Après un court instant, la vanne 54 est fermée puis les boîtes 20 sont alimentées en deuxième fluide F2 de façon séquentielle. Ainsi, les vannes 56 et 54 sont dans un premier temps fermées et la vanne 55 ouverte. Le flux de fluide F2 dans la première boîte 20-1 dure le temps nécessaire pour remplir une zone de culture cellulaire de la boîte 20-1 en deuxième fluide F2. Une fois la première boîte 20-1 alimentée en deuxième fluide F2, la vanne 55 est fermée, puis la vanne 56 ouverte, afin que le deuxième fluide F2 s'achemine maintenant dans la deuxième boîte 20-2. Une fois toutes les boîtes 20 remplies, la pompe 74 est arrêtée, et la vanne 56 fermée. Comme dans une étape ultérieure, le deuxième réseau fluidique d'alimentation 64 et de distribution 24 vont de nouveau recevoir un deuxième fluide F2, ceux-ci sont nettoyés. De ce fait, les vannes 82 et 54 sont ouvertes (la vanne 81 étant fermée) et sous l'action de la pompe 74, le liquide de rinçage 93 est acheminé dans le deuxième réseau fluidique d'alimentation 64 pour le nettoyer.

Dans une étape P6 d'incubation, l'installation 10 est alors mise au repos, avec le système de distribution 14 placé dans un incubateur. Les connexions 30, 32, 34 n'ont selon un mode de réalisation, pas besoin d'être déconnectées pour permettre au support 18 d'être placé dans l'incubateur.

Une fois l'incubation finie et avant l'acheminement d'un autre milieu de culture / deuxième fluide F2, les boîtes 20 sont drainées séquentiellement dans une étape P7. Ainsi, les vannes 81 et 54 sont fermées. Dans un premier temps, la vanne 55 est ouverte et la vanne 56 fermée. Sous l'action de la pompe 76, du fluide F3 présent dans la boîte première 20-1 est drainé vers la poche de vidange 73 préalablement connectée au troisième réseau fluidique d'alimentation 66 via la connexion aseptique 67. Une fois la première boîte 20-1 drainée, la valve 55 est fermée puis la valve 56 ouverte afin de maintenant drainer la deuxième boîte 20-2, et ainsi de suite pour chaque boîte 20.

Dans une étape P8 d'alimentation en deuxième fluide F2, une fois le drainage effectué, chacune des boîtes 20 est alimentée en deuxième fluide F2 séquentiellement comme décrit précédemment à l'étape P5, puis l'installation 10 est mise au repos le temps d'une incubation.

Les étapes P6, P7 et P8 sont répétées autant de fois qu'un deuxième fluide F2 est acheminé vers les boîtes 20.

L'installation 10 peut être contrôlée de façon numérique. Les différentes valves et pompes peuvent être contrôlées numériquement de sorte que la culture de tissu cellulaire se fasse de façon automatique avec un minimum d'apport humain. Un algorithme peut servir à programmer les séquences d'ouverture et fermeture des valves et pompes. Le contrôle de l'installation 10 peut se faire à distance.

Dans une étape P9 de désassemblage, une fois la culture terminée, les premier 22, deuxième 24, et troisième 26 réseaux de distribution sont déconnectés des premier 62, deuxième 64, et troisième 66 réseaux d'alimentation. Les boîtes 20 sont désolidarisées des premier 22, deuxième 24, et troisième 26 réseaux de distribution par soudure aseptique au niveau des connexions aseptiques 30, 32, 34. Les boîtes 20 peuvent alors être cryogénées, et transportées telles quelles. Elles peuvent être désolidarisées du support 18.

Les supports 18 (ou platines) sur lesquelles sont placées l'ensemble réseau fluidique de distribution 22, 24, 26 + boîtes 20 reposent sur un bâti de l'installation. Chaque boîte 20 peut être placée sur un support individuel mécanisé qui permet la mise en mouvement desdites boîtes afin d'effectuer une agitation transversale mais également une inclinaison (afin de faciliter la vidange de la boîte). L'agitation peut alternativement être commune à toutes les boîtes se trouvant sur un même support 18, de sorte que le support 18 soit agité.

En référence maintenant aux figures 3 à 11, une boîte 110 de culture et transport de tissu cellulaire TC selon un mode de réalisation de la boîte 20 va être décrite. La boîte 110 comprend un corps de boîte 112 ayant un intérieur 114 accessible et une ouverture supérieure 116. L'ouverture supérieure 116 est fermée par un moyen de fermeture 118. Le moyen de fermeture118 est préférentiellement étanche aux fluides et aux gaz. Selon une autre mode de réalisation, le filtre est faiblement perméable aux fluides et aux gaz. Un filtre est faiblement perméable si le taux de transmission de la vapeur d'eau est inférieur à 5g/m²/24h, de préférence 1g/m²/24h.

La boîte 110 comprend au moins une connexion fluidique aseptique 120 à travers le corps de boîte 112. Dans l'exemple de la figure 3, la boîte 110 inclut trois connexions fluidiques aseptiques 120 : deux connexions fluidiques d'entrée 122 de fluides de culture, et une connexion fluidique de sortie 124 de fluides. Les connexions fluidiques d'entrée 122 permettent d'acheminer des fluides, préférentiellement liquides, permettant la culture de tissu cellulaire TC, tandis que la connexion fluidique de sortie 124 permet de drainer la culture de tissu cellulaire TC de ces mêmes fluides. L'intérieur 114 du corps de boîte 112 inclut une zone (ou volume) de travail 136 adaptée à recevoir la culture de tissu cellulaire TC et les milieux fluides de culture permettant au tissu cellulaire TC de se développer.

La boîte 110 inclut de plus un filtre 126 disposé dans le corps de boîte 112 (c'est-à-dire dans la paroi délimitant l'intérieur de l'extérieur de la boîte 10). Le filtre 26 communique l'intérieur du corps de boîte 112 avec un extérieur 128 du corps de boîte 112. Le filtre 126 est configuré pour permettre un échange gazeux entre l'intérieur 114 et l'extérieur 128 du corps de boîte 121, tout en empêchant le passage de liquides.Le filtre 26 est configuré pour empêcher le passage de bactéries et de virus. Le filtre 26 est l'unique moyen de communication entre l'intérieur de l'extérieur de la boîte 10, en dehors des trois connexions fluidiques aseptiques 20.

Selon un mode de réalisation, le filtre 26 n'est pas disposé dans le corps de boîte 12 mais à l'extérieur 28 du corps de boîte 12. Il communiquerait avec l'intérieur 14 du corps boîte 12 grâce à un moyen de liaison, tel qu'un tube relié au corps de boîte 12. Positionner le filtre à l'extérieur 28 du corps de boîte 12 permet d'éviter les deltas de pression (de l'ordre de -0.5 bar) lors de la cryopréservation.

Selon un mode de réalisation, le filtre est hydrophobe.

La boîte 110 peut inclure optionnellement un insert 130 amovible disposé dans la zone de travail 136 et adapté à recevoir la culture de tissu cellulaire TC. L'insert 130 peut être inséré dans l'intérieur 114 du corps de boîte 112 à travers l'ouverture supérieure 116. De ce fait, l'ouverture supérieure 116 est dimensionnée pour permettre le passage de l'insert 130. L'insert 130 peut avoir une zone de culture 134 adaptée à recevoir et cultiver le tissu cellulaire TC et une zone de préhension 135 permettant de manipuler l'insert 130, notamment de le mettre et/ou enlever du corps de boîte 112. L'insert 130 sera décrit en plus de détails ci-dessous, en relation avec la figure 5. Selon d'autres modes de réalisation, la boîte 110 pourrait ne pas avoir d'insert, de sorte que la culture de tissu cellulaire TC soit directement faite sur l'intérieur 114 du corps de boîte 112 au niveau de la zone de travail 136.

La zone de travail 136 est dimensionnée de sorte à contenir un volume de liquide de culture adéquate. Dans le cas de l'insert 130, la zone de travail 136 a un volume légèrement supérieur à celui de la zone de culture 134 de l'insert 130.

La zone de travail 136 du corps de boîte 112 inclut une surface de travail 132 adaptée à recevoir la culture de tissu cellulaire TC. Dans le cas de boîtes ayant un insert, la surface de travail 132 est adaptée à recevoir la zone de culture 134 de l'insert 130. Dans le cas de boîtes 110 sans insert, le tissu cellulaire TC sera directement disposé sur la surface de travail 132 de la boîte 110. Dans ce dernier cas, la surface de travail 132 peut avoir subi un traitement de surface au plasma afin de permettre au tissu cellulaire TC de s'accrocher à la surface de travail 132. La surface de travail 132 pourrait alternativement avoir subi un traitement corona.

Que ce soit avec ou sans insert, la surface de travail 132 peut par exemple être transparente afin de permettre un contrôle visuel de la cuture de tissu cellulaire TC, sans avoir à ouvrir le moyen de fermeture 118. La surface de travail 132 peut faire partie du corps de boîte 112 délimitant l'intérieur 114 de l'extérieur 128. De façon plus large, tout ou partie du corps de boîte 112 pourrait être transparent. La surface de travail 132 est préférentiellement plate. Elle peut être un renforcement du corps de boîte 112, comme illustré dans les figures. La surface de travail 132 peut être détachable du corps de boîte 112 de sorte à extraire le tissu cellulaire TC lorsque celui-ci est formé, comme il sera expliqué en relation avec les figures 8 à 10.

Le moyen de fermeture 118 sert à réaliser un système clos dans la boîte 110 qui permet la culture de tissu cellulaire TC. Le moyen de fermeture 118 peut être scellé au corps de boîte 112, par exemple par thermoscellage ou ultrasons. Le moyen de fermeture 118 peut inclure une languette 119 qui permet de retirer le moyen de fermeture 118 à la main sans avoir recours à des instruments, comme par exemple un scalpel.

Le moyen de fermeture 118 peut être à usage unique de sorte qu'une fois désolidarisé de l'ouverture supérieure, 116 l'intérieur 114 du corps de boîte 112 est accessible par l'ouverture supérieure 116. Ainsi, la boîte 110 de culture et transport de tissu cellulaire peut être un produit à usage unique. Dans l'exemple des figures, le moyen de fermeture 118 est transparent afin de permettre un contrôle visuel de la culture de tissu cellulaire. Il se pourrait que le moyen de fermeture 118 ne soit pas transparent, ou bien partiellement transparent, de couleur ou clair. Afin de supporter la cryogénie lors du transport et de la conservation de la culture de tissu cellulaire, le moyen de fermeture 118 est préférentiellement adapté à conserver ses propriétés mécaniques jusqu'à une température d'exposition d'environ -200° C. Le moyen de fermeture 118 est par exemple fait de polyethylene terephthalate ou polyethylène.

Le corps de boîte 112 peut être réalisé par injection plastique. Le corps de boîte 112 peut être par exemple fait de polypropylène, éthylène propylène fluoré, polycarbonate, ou Eastman Tritan^{™} Copolyester MP100. Le corps de boîte 112 peut être adapté à conserver ses propriétés mécaniques jusqu'à une température d'exposition d'environ -200° C, afin de permettre la cryogénie de la boîte 110. Le corps de boîte 112 peut être résistant au dimethylsulfoxide (DMSO). Le corps de boîte 112 peut être substantiellement parallélépipédique à bords arrondis afin de faciliter le transport sans pertes de place. Le corps de boîte 112 peut avoir une forme généralement ovoïde vue du dessus.

Le corps de boîte 112 peut ainsi inclure un renforcement 113 permettant de recevoir une extrémité de la zone de préhension 135 de l'insert 130 afin que l'insert 130 ne dépasse de la boîte 110.

L'intérieur 114 du corps de boîte 112 peut inclure une partie inclinée 138 vers la surface de travail 132. La partie inclinée 138 peut permettre l'écoulement des fluides vers la zone de culture 136 de tissu cellulaire TC. L'inclinaison de la partie inclinée 138 est en référence à un plan horizontal H, le plan horizontal H contenant la surface de travail 132. La partie inclinée 138 est selon un mode de réalisation en regard des connexions fluidiques 120.

La partie inclinée 138 peut recevoir le filtre 126. Le filtre 126 peut être disposé horizontalement dans la partie inclinée 138 ou bien être incliné, par exemple en ayant la même inclinaison que celle de la partie inclinée comme illustré aux figures 3 à 7. Le filtre 126 pourrait être disposé verticalement dans la partie inclinée 138 comme illustré par le filtre 126' de la figure 11. Un canal d'évaluation C pourrait alors s'étendre à partir de la partie inclinée 138 à travers le filtre 126' et vers l'extérieur de la boîte. Selon un autre mode de réalisation, le filtre 126 est disposé au niveau des connexions fluidiques 120. Le filtre 126 peut être disposé dans le corps de boîte 112 distalement de la surface de travail 132. Ceci permet de limiter les risques d'humidification du filtre 126. Selon un mode de réalisation, et comme montré dans les figures, le filtre 126 peut être disposé verticalement en dessous de la zone de préhension 135 de l'insert 130. Le filtre 126 peut être est fait de polyéthylene, polyuréthane, polyester ou polypropylène. Afin de supporter la cryogénie lors du transport et de la conservation de la culture de tissu cellulaire TC, le filtre 126 est préférentiellement adapté à conserver ses propriétés mécaniques jusqu'à une température d'exposition d'environ -200° C. La boîte 110 pourrait inclure plus d'un filtre.

Selon un autre mode de réalisation, le filtre 126 est disposé dans le corps de boîte 112 au niveau des connexion fluidiques 120. Selon un mode de réalisation, le filtre 26 est disposé sur le corps de boîte 112 à l'opposé des connexions fluidiques 120. Cette dernière configuration peut permettre de limiter les risques d'humidification du filtre 126.

Les deux connexions fluidiques d'entrée 122 de fluides sont des ports ayant une extension tubulaire intérieure 140 s'étendant vers l'intérieur 114 du corps de boîte 112. Chaque extension tubulaire 140 a une extrémité qui se trouve verticalement au-dessus de la surface de travail 132, ou si la boîte a un insert 130, verticalement au-dessus de la zone de culture 134 de l'insert 130. Les extensions tubulaires intérieures 140 sont dimensionnées pour être assez longues de sorte à alimenter le milieu de culture, mais en même temps pas trop longues de sorte à ne gêner l'enlèvement de l'insert 130. Selon un mode de réalisation, une extrémité 142 des connexions fluidiques d'entrée 122 dans l'intérieur 114 du corps de boîte 112 est en biseau. La forme biseautée permet un écoulement vers la zone de culture 34/surface de travail 132. Le biseau est par exemple orienté en direction de l'ouverture supérieure 116. Le biseau peut être d'environ 45 degrés. Les extensions tubulaires intérieures 140 peuvent être scellées au corps de boîte 112 par soudure et les biseaux obtenus à l'aide d'une machine de déconnexion aseptique.

Les connexions fluidiques 120 ont aussi chacune une extension tubulaire extérieure 141 s'étendant à partir de l'extérieur du corps de boîte 112 de sorte à connecter à des tubes T contenant des liquides de culture ou de drainage (montrés en pointillés). Les extensions tubulaires extérieures 141 sont adaptés à connecter aux tubes T par exemple par un système male femelle avec un serflex qui vient assurer l'étanchéité, un système mécanique, par soudure ultrason ou en utilisant de la colle biocompatible.

Les connexions fluidiques d'entrée 122 et/ou la connexion fluidique de sortie 124 de fluides peut être un septum permettant d'être percés par une aiguille tout en garantissant l'étanchéité. Les connexions fluidiques d'entrée 122 et/ou la connexion fluidique de sortie 124 de fluides peut être résistante au dimethylsulfoxide (DMSO) ou tout autre milieu de préservation injecté dans la boîte en fin de processus de culture cellulaire.

La boîte 110 pourrait aussi avoir des fixations externes, par exemple une fente dans l'extérieur du corps de boîte 112, afin de permettre de fixer temporairement la boîte sur un support permettant l'alimentation de la boîte en milieux de culture. Le support peut ainsi contenir une pluralité de boîtes 110 pour assurer une production en parallèle du tissu cellulaire TC.

Comme mentionné précédemment, l'insert amovible 130 comprend la zone de culture 134 adaptée à recevoir et cultiver le tissu cellulaire TC et la zone de préhension 135 permettant de manipuler l'insert 130, notamment de le mettre et/ou enlever du corps de boîte 112. Cette manipulation peut se faire grâce aux bouts de doigts, ou si la boîte 110 le permet (notamment de par la taille de la cavité se trouvant sous la zone de préhension 135 et visible à la figure 4 de par la partie inclinée 138) avec un bout des phalanges.

L'insert 130 (la zone de culture 134 et zone de préhension 135) peut être fait de polyester, polypropylène, éthylène propylène fluoré, polycarbonate.

La zone de culture 134 peut inclure une ouverture supérieure 144 adaptée à accueillir la culture de tissu cellulaire TC afin de nourrir les cellules par le dessus, notamment par les connexions fluidiques d'entrée 122. La zone de culture 134 inclut un fond poreux 146 en regard de l'ouverture supérieure 116 lorsque l'insert 130 est disposé dans le corps de boîte 112 sur la surface de travail 132. Le fond poreux 146 de l'insert 130 permet de recevoir la culture de tissu cellulaire TC. Il est poreux afin de permettre au milieu de culture fluides d'imbiber le dessus et le dessous de la culture de tissu cellulaire TC. Le fond poreux 146 peut être fait de polyéthylène ou polycarbonate. Le fond poreux 146 peut être transparent. Le fond poreux 146 pourrait être flexible ou rigide. Il pourrait avoir subi un traitement au plasma. Par poreux on entend que le fond permet l'évacuation de liquides que ce soit à travers un réseau de cellules ouvertes ou un ou plusieurs trous d'évacuation. Le fond poreux 146 pourrait être détachable ou pas du reste de l'insert 130. Selon un mode de réalisation, la taille des pores du fond poreux est de 0,2 à 4µm, de préférence de 0,2 à 0,6µm. Selon un mode de réalisation, la densité des pores du fond poreux est de 1*10⁶ à 5*10⁶, de préférence de 1*10⁶ à 3*10⁶ pores par cm².

La zone de culture 134 de l'insert 130 est de forme généralement arrondie, par exemple en forme d'anneau. La zone de culture 134 de l'insert 130 peut avoir une épaisseur E inférieure ou correspondant à une épaisseur de la surface de travail 138 du corps de boîte 112 adaptée à recevoir la zone de culture 134 de l'insert 130. Ceci peut permettre un bon écoulement des fluides. Selon un mode de réalisation, la zone de culture 134 de l'insert 130 a une forme correspondant à une forme de la surface de travail 132 du corps de boîte 112 adaptée à recevoir la zone de culture 134 de l'insert 130 pour éviter que l'insert 130 ne bouge vis-à-vis du corps de boîte 112 lors du transport.

La zone de préhension 134 est selon un mode de réalisation et tel qu'illustré dans les figures, un manche. La zone de préhension 134 pourrait cependant avoir toute forme permettant de manipuler l'insert 130. Dans le mode de réalisation illustré dans les figures, la zone de préhension 134 est disposée en regard de la partie inclinée 138. La zone de préhension 134 peut avoir une inclinaison par rapport au plan horizontal H en direction de la surface de culture 132 afin de permettre l'écoulement de fluides condensés par exemple. L'inclinaison de la zone de préhension 134 par rapport au plan horizontal H peut être substantiellement l'inclinaison de la partie inclinée 138.

L'insert 130 peut inclure de plus une (ou plusieurs) connexion 147 adaptée à coopérer de manière amovible avec l'intérieur 114 du corps de boîte 112. Les connexions 147 peuvent permettre d'éviter que l'insert 30 ne bouge vis-à-vis du corps de boîte 112 lors du transport. Selon un mode de réalisation, et tel qu'illustré dans les figures, les connexions 147 sont des pattes adaptées à être insérée de manière amovible dans un logement 148 de l'intérieur 114 du corps de boîte 112. Le logement 148 peut être un renfoncement ouvert afin de retirer l'insert 130 vers le haut.

L'insert 130 peut ne pas avoir les connexions 147. En revanche, l'insert 130 peut avoir des pattes 149 (2 ou plus) disposées sur les bords latéraux de l'insert 130 avec par exemple des creux au niveau des pattes pour poser et glisser l'insert 130. Les pattes 149 sont illustrées à la figure 7 en pointillés. Les pattes1 49 permettent de diminuer la quantité de milieu de culture nécessaire pour la culture cellulaire.

L'insert 130 peut être fait de polypropylène, éthylène propylène fluoré, ou polycarbonate. L'insert 130 peut être résistant au dimethylsulfoxide (DMSO).

La boîte 110 est utilisable comme-ci. Dans un premier temps la boîte 110 est livrée avec le moyen de fermeture 118 scellé, et l'insert 130 à l'intérieur 114 de la boîte 110, si la boîte 110 comporte un tel insert. L'insert 130 peut contenir des cellules dans la zone de travail 136 (c'est à sire sur la surface de travail 132 si la boîte n'a pas d'insert, ou sur le fond poreux 146 si la boîte a un insert) qui serviront à cultiver le tissu cellulaire TC. La boîte 110 est alors connectée via les connexions fluides 120 à des milieux de culture par les tubes T. Il peut y avoir un ou plusieurs milieux de culture qui sont acheminés dans la zone de travail 36 par les connexions fluidiques d'entrée 122, de façon séquentielle, avec (ou pas) des temps de repos entre. La connexion fluidique de sortie 124 permet de drainer la zone de travail 136 entre les différents acheminements de milieux de culture. La culture du tissu cellulaire TC se fait donc dans un milieu fermé avec peu de risques de contamination. Lorsque le tissu cellulaire TC est créé, la boîte 110 peut être cryogénée et transportée. Lorsque l'opérateur décide d'utiliser le tissu cellulaire TC, il peut le récupérer de plusieurs manières. Il peut ouvrir le moyen de fermeture 118 et récupérer le tissu cellulaire TC. Si la boîte 110 possède l'insert, il peut alors retirer l'insert par l'ouverture supérieure 138 ainsi dégagée du moyen de fermeture 118.

Selon un mode de réalisation, et comme illustré à la figure 9, le fond poreux 146 de l'insert 130 peut être détachable, au besoin via une languette, de sorte à extraire le tissu cellulaire TC de l'insert 130 sans l'endommager. Dans un premier temps, le fond poreux 146 avec le tissu cellulaire TC dessus peut être désolidarisé de l'insert 130, puis le fond poreux 146 enlevé du tissu cellulaire TC de sorte à pourvoir appliquer le tissu cellulaire TC seul, par exemple, sur la peau du patient S.

Selon un autre mode de réalisation et tel qu'illustré à la figure 9, le fond poreux 146 est dissocié de l'insert 130 et le tissu cellulaire TC reste pour un temps accroché à l'insert 130. L'insert 30 est alors appliqué au niveau de la peau S du patient afin de poser le tissu cellulaire TC sur la peau S du patient sans le manipuler directement.

D'autre part, la boîte 10 pourrait avoir un fond amovible pour récupérer le tissu cellulaire TC, notamment lorsque la boîte 110 n'a pas d'insert. En relation avec la figure 10, la surface de travail 132 du corps de boîte 112 est amovible, et est de préférence souple. Lorsque le tissu cellulaire TC est formé et est prêt à être utilisé, la surface de travail 132 est alors enlevée du corps de boîte 112 et avec elle le tissu cellulaire TC. Le tissu cellulaire TC est alors enlevé de la surface de travail 132 et déposé sur la peau S du patient.

En référence maintenant à la figure 12, un autre mode de réalisation de la boîte 110, que nous appellerons boîte 210 va être décrit. La boîte 210 comprend tous les éléments et modes de réalisation de la boîte 110, qui ne seront pas décrits de nouveau et pour lesquels des références numériques similaires mais dans la deux centaine seront utilisées.

La boîte 210 se distingue de la boîte 110 par le fait que le corps de boîte 212 comprend une **cloison 250 qui** sépare la zone de travail 236 du filtre 226. Plus précisément, dans le mode de réalisation des figures, la cloison 250 se trouve entre la zone de travail 236 et la partie inclinée 238 à de l'intérieur 214 du corps de boîte 212. À ce titre, et comme il est illustré dans la figure 12, dans ce mode de réalisation, la partie 238 peut ne pas être inclinée. Elle est aussi positionnée en position plus basse que la partie 238 de sorte à éviter que la cloison 250 touche le moyen de fermeture 218 fermant l'ouverture supérieure 216. De façon générale, la cloison 250 est verticale et ne touche pas le moyen de fermeture 218 fermant l'ouverture supérieure 216 du corps de boîte 212. La cloison 250 permet de sécuriser le filtre et éviter qu'il ne soit mouillé lors des manipulations de la boîte et lors des phases d'agitation.

## Revendications

1. **Installation (10) de culture de tissu cellulaire** comprenant :
un **système de distribution (12)** comprenant :
- un **support (18)** adapté à recevoir au moins deux **boîtes (20, 20-1, 20-2)** de culture de tissu cellulaire,
- un **premier réseau fluidique de distribution (22)** connecté au support (18), le premier réseau fluidique de distribution (22) étant adapté à amener un **premier fluide (F1)** de type biomatériau vers chacune des boîtes (20, 20-1, 20-2) de culture de tissu cellulaire, le premier réseau fluidique de distribution (22) ayant un premier **port aseptique (30) d'entrée** sur le support (18), le premier port aseptique d'entrée étant un unique port d'entrée pour le premier fluide (F1),
- un **deuxième réseau fluidique de distribution (24)** connecté au support (18), le deuxième réseau fluidique de distribution (24) étant adapté à amener un **deuxième fluide (F2)** vers chacune des boîtes (20, 20-1, 20-2) de culture de tissu cellulaire, le deuxième réseau fluidique de distribution (24) ayant un deuxième **port aseptique (32) d'entrée** sur le support (18) différent du premier port aseptique d'entrée, le deuxième port aseptique d'entrée étant un unique port d'entrée pour le deuxième fluide (F2),
- un **troisième réseau fluidique de distribution (26)** connecté au support (18), le troisième réseau fluidique de distribution (26) étant adapté à vidanger un **troisième fluide (F3)** de chacune des boîtes (20, 20-1, 20-2) de culture de tissu cellulaire, le troisième réseau fluidique de distribution (26) ayant un unique **port aseptique (34) de sortie** sur le support (18), et
un **système d'alimentation (14)** connecté au système de distribution (12), le système d'alimentation (14) comprenant :
- un **premier réseau fluidique d'alimentation (62)** connecté aseptiquement au premier réseau fluidique de distribution (22) via le premier port aseptique (30) d'entrée, le premier réseau fluidique d'alimentation (62) inclut une **connexion aseptique (63)** adaptée à connecter avec une **première poche (70)** lors de leur mélange dans le mélangeur statique polymérisent pour former le premier fluide (F1), le premier réseau fluidique d'alimentation (62) incluant une pompe (72, 72-1, 72-2) adaptée à amener le premier fluide (F1) vers le premier réseau fluidique de distribution (22),
- un **deuxième réseau fluidique d'alimentation (64)** connecté aseptiquement au deuxième réseau fluidique de distribution (24) via le deuxième port aseptique (32) d'entrée, le deuxième réseau fluidique optionnel d'alimentation (64) inclut une **connexion aseptique (65)** adaptée à connecter avec une **deuxième poche (71)** contenant le deuxième fluide (F2), un deuxième réseau fluidique d'alimentation (64) incluant une pompe (74) adaptée à amener le deuxième fluide (F2) vers le deuxième réseau fluidique de distribution (24), et
- un **troisième réseau fluidique d'alimentation (66)** connecté aseptiquement au troisième réseau fluidique de distribution (26) via le port aseptique (34) de sortie, le troisième réseau fluidique d'alimentation (66) inclut une **connexion aseptique (67)** adaptée à connecter avec une **poche de vidange (73),** le troisième réseau fluidique d'alimentation (66) incluant une pompe (76) adaptée à vidanger le troisième fluide (F3) du troisième réseau fluidique de distribution (26).

2. Installation (10) selon la revendication 1, dans laquelle le support (18) inclut de plus un **système de vidange (60)** connecté à au moins un des du premier (22) et optionnellement deuxième (24) réseaux fluidiques de distribution.

3. Installation (10) selon la revendication 1 ou 2, dans laquelle chacun des premier (22), optionnellement deuxième (24) et troisième (26) réseaux fluidiques de distribution comprend une **connexion aseptique (30, 32, 34)** adaptée à connecter chacun des premier (22), optionnellement deuxième (24) et troisième (26) réseaux fluidiques de distribution aux boîtes (20, 20-1, 20-2) de culture de tissu cellulaire.

4. Installation (10) selon l'une des revendications précédentes, dans laquelle chacun des premier (22), optionnellement deuxième (24) et troisième (26) réseaux fluidiques de distribution inclut un **tube principal (40, 44, 47)** et deux **tubes secondaires (41, 42, 45, 46, 48, 49),** le tube principal (40, 44, 47) étant d'une part connecté au port aseptique (30, 32, 34) et d'autre part connecté aux tubes secondaires (41, 42, 45, 46, 48, 49), chacun des tubes secondaires étant connecté à une boîte de culture de tissu cellulaire associée (20, 20-1, 20-2).

5. Installation (10) selon l'une des revendications précédentes, dans lequel les pompes de chacun des premier (62), deuxième (64) et troisième (66) réseau fluidique d'alimentation est une pompe péristaltique.

6. Installation (10) selon l'une des revendications précédentes, comprenant de plus un **système de rinçage (90)** connecté au premier ou deuxième réseau fluidique d'alimentation (64).

7. Installation (10) selon l'une des revendications précédentes, dans laquelle le premier (62) réseau fluidique d'alimentation comprend un **mélangeur statique (89)** adapté à produire le premier fluide (F1) après passage dans le mélangeur statique.

8. Installation (10) selon la revendication précédente, dans laquelle la **connexion aseptique (63)** connecte avec deux **poches (70-1, 70-2)** contenant chacune un **composant fluide (F1-1, F1-2)** qui se polymérisent lors du mélange par le mélangeur statique (89) pour former le premier fluide (F1).

9. Installation (10) selon l'une des revendications précédentes, dans laquelle le système de distribution (14) est un premier système de distribution, et comprenant de plus un deuxième système de distribution, le deuxième système de distribution ayant deux ports d'entrée aseptique et un port de sortie aseptique sur le support, chacun des premier (62), deuxième (64) et troisième (66) réseaux fluidiques d'alimentation ayant une première connexion aseptique au premier système de distribution et une deuxième connexion aseptique au deuxième système de distribution.

10. Installation (10) selon l'une des revendications précédentes, comprenant de plus un support individuel mécanisé pour chaque boîte, le support individuel mécanisé permettant la mise en mouvement des boîtes afin d'effectuer une agitation transversale mais également une inclinaison.

## Patentansprüche

1. Anlage (10) zur Kultur von Zellgewebe, umfassend:
ein Verteilungssystem (12), umfassend:
- einen Träger (18), der dazu ausgelegt ist, mindestens zwei Zellgewebekulturboxen (20, 20-1, 20-2) aufzunehmen,
- ein erstes Fluidverteilungsnetz (22), das mit dem Träger (18) verbunden ist, wobei das erste Fluidverteilungsnetz (22) dazu ausgelegt ist, jeder der Zellgewebekulturboxen (20, 20-1, 20-2) ein erstes Fluid (F1) vom Typ Biomaterial zuzuführen, wobei das erste Fluidverteilungsnetz (22) einen ersten aseptischen Einlassanschluss (30) an dem Träger (18) aufweist, wobei der erste aseptische Einlassanschluss ein einziger Einlassanschluss für das erste Fluid (F1) ist,
- ein zweites Fluidverteilungsnetz (24), das mit dem Träger (18) verbunden ist, wobei das zweite Fluidverteilungsnetz (24) dazu ausgelegt ist, jeder der Zellgewebekulturboxen (20, 20-1, 20-2) ein zweites Fluid (F2) zuzuführen, wobei das zweite Fluidverteilungsnetz (24) einen zweiten aseptischen Einlassanschluss (32) an dem Träger (18) aufweist, der sich von dem ersten aseptischen Einlassanschluss unterscheidet, wobei der zweite aseptische Einlassanschluss ein einziger Einlassanschluss für das zweite Fluid (F2) ist,
- ein drittes Fluidverteilungsnetz (26), das mit dem Träger (18) verbunden ist, wobei das dritte Fluidverteilungsnetz (26) dazu ausgelegt ist, ein drittes Fluid (F3) aus jeder der Zellgewebekulturboxen (20, 20-1, 20-2) abzulassen, wobei das dritte Fluidverteilungsnetz (26) einen einzigen aseptischen Auslassanschluss (34) an dem Träger (18) aufweist, und ein Versorgungssystem (14), das mit dem Verteilungssystem (12) verbunden ist, das Versorgungssystem (14) umfassend:
- ein erstes Fluidversorgungsnetz (62), das über den ersten aseptischen Einlassanschluss (30) aseptisch mit dem ersten Fluidverteilungsnetz (22) verbunden ist, wobei das erste Fluidversorgungsnetz (62) eine aseptische Verbindung (63) beinhaltet, die dazu ausgelegt ist, mit einem ersten Beutel (70) verbunden zu werden, bei ihrem Mischen in dem statischen Mischer polymerisieren, um das erste Fluid (F1) zu bilden, wobei das erste Fluidversorgungsnetz (62) eine Pumpe (72, 72-1, 72-2) beinhaltet, die dazu ausgelegt ist, dem ersten Fluidverteilungsfluidnetz (22) das erste Fluid (F1) zuzuführen,
- ein zweites Fluidversorgungsnetz (64), das über den zweiten aseptischen Einlassanschluss (32) aseptisch mit dem zweiten Fluidverteilungsnetz (24) verbunden ist, wobei das optionale zweite Fluidverteilungsnetz (64) eine aseptische Verbindung (65) beinhaltet, die dazu ausgelegt ist, mit einem zweiten Beutel (71) verbunden zu werden, der das zweite Fluid (F2) enthält, wobei ein zweites Fluidversorgungsnetz (64) eine Pumpe (74) beinhaltet, die dazu ausgelegt ist, dem zweiten Fluidverteilungsnetz (24) das zweite Fluid (F2) zuzuführen, und
- ein drittes Fluidversorgungsnetz (66), das über den aseptischen Auslassanschluss (34) aseptisch mit dem dritten Fluidverteilungsnetz (26) verbunden ist, wobei das dritte Fluidversorgungsnetz (66) eine aseptische Verbindung (67) beinhaltet, die dazu ausgelegt ist, mit einem Abflussbeutel (73) verbunden zu werden, wobei das dritte Fluidversorgungsnetz (66) eine Pumpe (76) beinhaltet, die dazu ausgelegt ist, das dritte Fluid (F3) aus dem dritten Fluidverteilungsnetz (26) abzulassen.

2. Anlage (10) nach Anspruch 1, wobei der Träger (18) ferner ein Ablasssystem (60) beinhaltet, das mit mindestens einem von den ersten (22) und optional zweiten (24) Fluidverteilungsnetzen verbunden ist.

3. Anlage (10) nach Anspruch 1 oder 2, wobei jedes der ersten (22), optional zweiten (24) und dritten (26) Fluidverteilungsnetze eine aseptische Verbindung (30, 32, 34) umfasst, die dazu ausgelegt ist, jedes der ersten (22), optional zweiten (24) und dritten (26) Fluidverteilungsnetze mit den Zellgewebekulturboxen (20, 20-1, 20-2) zu verbinden.

4. Anlage (10) nach einem der vorhergehenden Ansprüche, wobei jedes der ersten (22), optional zweiten (24) und dritten (26) Fluidverteilungsnetze ein Hauptrohr (40, 44, 47) und zwei Nebenrohre (41, 42, 45, 46, 48, 49) beinhaltet, wobei das Hauptrohr (40, 44, 47) zum einen mit dem aseptischen Anschluss (30, 32, 34) verbunden ist und zum anderen mit den Nebenrohren (41, 42, 45, 46, 48, 49) verbunden ist, wobei jedes der Nebenrohre mit einer zugeordneten Zellgewebekulturbox (20, 20-1,20-2) verbunden ist.

5. Anlage (10) nach einem der vorhergehenden Ansprüche, wobei die Pumpen jedes der ersten (62), zweiten (64) und dritten (66) Fluidversorgungsnetze eine Peristaltikpumpe ist.

6. Anlage (10) nach einem der vorhergehenden Ansprüche, umfassend ferner ein Spülsystem (90), das mit dem ersten oder zweiten Fluidversorgungsnetz (64) verbunden ist.

7. Anlage (10) nach einem der vorhergehenden Ansprüche, wobei das erste Fluidversorgungsnetz (62) einen statischen Mischer (89) umfasst, der dazu ausgelegt ist, das erste Fluid (F1) nach dem Durchgang durch den statischen Mischer zu erzeugen.

8. Anlage (10) nach dem vorhergehenden Anspruch, wobei die aseptische Verbindung (63) mit zwei Beuteln (70-1, 70-2) verbunden wird, die jeweils eine Fluidkomponente (F1-1, F1-2) enthalten, die beim Mischen durch den statischen Mischer (89) polymerisieren, um das erste Fluid (F1) zu bilden.

9. Anlage (10) nach einem der vorhergehenden Ansprüche, wobei das Verteilungssystem (14) ein erstes Verteilungssystem ist, und umfassend ferner ein zweites Verteilungssystem, wobei das zweite Verteilungssystem zwei aseptische Einlassanschlüsse und einen aseptischen Auslassanschluss an dem Träger aufweist, wobei jedes der ersten (62), zweiten (64) und dritten (66) Fluidversorgungsnetze eine erste aseptische Verbindung mit dem ersten Verteilungssystem und eine zweite aseptische Verbindung mit dem zweiten Verteilungssystem aufweist.

10. Anlage (10) nach einem der vorhergehenden Ansprüche, umfassend ferner einen mechanisierten individuellen Träger für jede Box, wobei der mechanisierte individuelle Träger das Inbewegungversetzen der Boxen ermöglicht, um eine Querbewegung, aber auch eine Neigung durchzuführen.

## Claims

1. Cell tissue culture installation (10) comprising:
a distribution system (12) comprising:
- a support (18) designed to receive at least two cell tissue culture dishes (20, 20-1, 20-2),
- a first fluid distribution network (22) connected to the support (18), the first fluid distribution network (22) being designed to bring a first fluid (F1) of the biomaterial type to each of the cell tissue culture dishes (20, 20-1, 20-2), the first fluid distribution network (22) having a first aseptic inlet port (30) on the support (18), the first aseptic inlet port being a single inlet port for the first fluid (F1),
- a second fluid distribution network (24) connected to the support (18), the second fluid distribution network (24) being designed to feed a second fluid (F2) to each of the cell tissue culture dishes (20, 20-1, 20-2), the second fluid distribution network (24) having a second aseptic inlet port (32), different from the first aseptic inlet port, on the support (18), the second aseptic inlet port being a single inlet port for the second fluid (F2),
- a third fluid distribution network (26) connected to the support (18), the third fluid distribution network (26) being designed to drain a third fluid (F3) out of each of the cell tissue culture dishes (20, 20-1, 20-2), the third fluid distribution network (26) having a single aseptic outlet port (34) on the support (18), and
a supply system (14) connected to the distribution system (12), the supply system (14) comprising:
- a first fluid supply network (62) aseptically connected to the first fluid distribution network (22) via the first aseptic inlet port (30), the first fluid supply network (62) includes an aseptic connection (63) designed for connection to a first pouch (70) during their mixture in the static mixer polymerize to form the first fluid (F1), the first fluid supply network (62) including a pump (72, 72-1, 72-2) designed to feed the first fluid (F1) to the first fluid distribution network (22),
- a second fluid supply network (64) aseptically connected to the second fluid distribution network (24) via the second aseptic inlet port (32), the optional second fluid supply network (64) includes an aseptic connection (65) designed for connection to a second pouch (71) containing the second fluid (F2), a second fluid supply network (64) including a pump (74) designed to feed the second fluid (F2) to the second fluid distribution network (24), and
- a third fluid supply network (66) aseptically connected to the third fluid distribution network (26) via the aseptic outlet port (34), the third fluid supply network (66) includes an aseptic connection (67) designed for connection to a drainage pouch (73), the third fluid supply network (66) including a pump (76) designed to drain the third fluid (F3) out of the third fluid distribution network (26).

2. Installation (10) according to Claim 1, wherein the support (18) further includes a drainage system (60) connected to at least one of the first (22) and optionally second (24) fluid distribution networks.

3. Installation (10) according to Claim 1 or 2, wherein each of the first (22), optionally second (24) and third (26) fluid distribution networks comprises an aseptic connection (30, 32, 34) designed to connect each of the first (22), optionally second (24) and third (26) fluid distribution networks to the cell tissue culture dishes (20, 20-1, 20-2).

4. Installation (10) according to one of the preceding claims, wherein each of the first (22), optionally second (24) and third (26) fluid distribution networks includes a main tube (40, 44, 47) and two secondary tubes (41, 42, 45, 46, 48, 49), the main tube (40, 44, 47) being connected both to the aseptic port (30, 32, 34) and to the secondary tubes (41, 42, 45, 46, 48, 49), each of the secondary tubes being connected to an associated cell tissue culture dish (20, 20-1, 20-2).

5. Installation (10) according to one of the preceding claims, wherein the pumps of each of the first (62), second (64) and third (66) fluid supply networks is a peristaltic pump.

6. Installation (10) according to one of the preceding claims, further comprising a rinsing system (90) connected to the first or second fluid supply network (64).

7. Installation (10) according to one of the preceding claims, wherein the first (62) fluid supply network comprises a static mixer (89) designed to produce the first fluid (F1) after passage through the static mixer.

8. Installation (10) according to the preceding claim, wherein the aseptic connection (63) connects to two pouches (70-1, 70-2) each containing a fluid component (F1-1, F1-2) which polymerize during the mixing by the static mixer (89) to form the first fluid (F1).

9. Installation (10) according to one of the preceding claims, wherein the distribution system (14) is a first distribution system, and further comprising a second distribution system, the second distribution system having two aseptic inlet ports and one aseptic outlet port on the support, each of the first (62), second (64) and third (66) fluid supply networks having a first aseptic connection to the first distribution system and a second aseptic connection to the second distribution system.

10. Installation (10) according to one of the preceding claims, further comprising a mechanized individual support for each dish, the mechanized individual support allowing the dishes to be set in motion in order to transversely agitate but also incline them.
